# EUROPÄISCHE PATENTANMELDUNG

(11) **EP 0 810 203 A1**
(43) Veröffentlichungstag der Anmeldung: **03.12.1997**
(21) Anmeldenummer: 97105724.5
(22) Anmeldetag: 07.04.1997
(51) Int. Cl.: C07C 237/04, C08K 5/20, C09J 175/04, C07C 237/06

(54) **Ester- und amidgruppenhaltige Polyamine sowie ein Verfahren zu ihrer Herstellung und deren Verwendung**

(30) Priorität: 31.05.1996 DE 19621871
(71) Anmelder: HÜLS AKTIENGESELLSCHAFT, 45764 Marl (DE)
(72) Erfinder: Wolf, Elmar, Dr., 45661 Recklinghausen (DE)

(57) **Zusammenfassung**

Ester- und amidgruppenhaltige Polyamine, dadurch gekennzeichnet, daß sie mindestens eine Amidgruppe enthalten und folgende Zusammensetzung besitzen 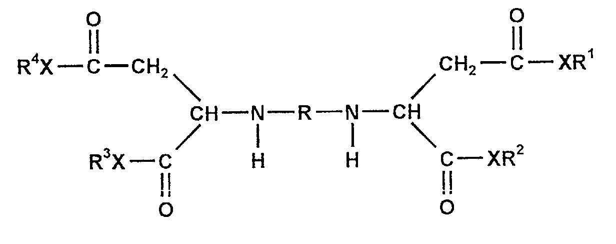 und die Substituenten die nachstehende Bedeutung haben:
- R: ist ein linearer, verzweigter oder cyclischer Kohlenwasserstoffrest mit 4 bis 18 C-Atomen,
- R¹, R², R³, R⁴: sind gleiche oder verschiedene lineare, verzweigte oder cyclische Alkylreste oder gleich Wasserstoff,
- X: repräsentiert 0 bis 3 Sauerstoffatome und 1 bis 4 Stickstoffatome.

## Beschreibung

Die vorliegende Erfindung betrifft neue ester- und amidgruppenhaltige Polyamine sowie ein Verfahren zu ihrer Herstellung und deren Verwendung.

Die Herstellung von Reaktionsprodukten aus Diaminen und zwei Molen Maleinsäureester wird in der DE-OS 16 70 812 als Zwischenstufe bei der Herstellung von Hydantoinen beschrieben. Die Verwendung dieser Asparaginsäure-Derivate (PADE abgekürzt) als Reaktionskomponente von Polyisocyanaten zur Herstellung von Überzügen erfolgt in der EP 0 403 921; in der EP 0 470 461 wird die Verwendung dieser PADE (+ Polyisocyanate) als Zweikomponenten-Bindemittelkombination in Autoreparaturlacken beansprucht. Man findet keinen Hinweis in der Literatur über die Verwendung dieser Asparaginsäure-Derivate zur Herstellung von 2K-PUR-Klebstoffen. Wie eigene Versuche zeigen, weisen Verklebungen, die mit diesen PADE und Polyisocyanaten hergestellt wurden, nur mäßige Zugscherfestigkeiten auf.

Überraschenderweise können diese Zugscherfestigkeiten beträchtlich erhöht werden, wenn die zur Verklebung eingesetzten Asparaginsäure-Derivate mindestens eine Amidgruppe enthalten.

Gegenstand der vorliegenden Erfindung sind somit ester- und amidgruppenhaltige Polyamine, durch gekennzeichnet, daß sie mindestens eine Amidgruppe enthalten und folgende Zusammensetzung besitzen 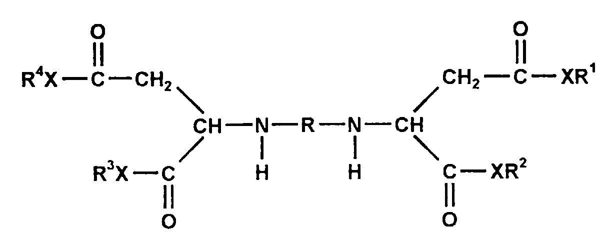 und die Substituenten die nachstehende Bedeutung haben:
- R: ist ein linearer, verzweigter oder cyclischer Kohlenwasserstoffrest mit 4 bis 18 C-Atomen,
- R¹, R², R³, R⁴: sind gleiche oder verschiedene lineare, verzweigte oder cyclische Alkylreste oder gleich Wasserstoff,
- X: repräsentiert 0 bis 3 Sauerstoffatome und 1 bis 4 Stickstoffatome.

Die cyclischen Kohlenwasserstoffreste der Substituenten können ebenfalls am Ring weitere Alkylgruppen wie z. B. Methyl, Ethyl, Propyl, Butyl, aufweisen. Des weiteren können CH₂-Gruppen der Substituenten R, R¹, R², R³ oder R⁴ durch Sauerstoffatome ersetzt werden.

Die erfindungsgemäßen Verbindungen zeichnen sich durch einen basischen Amingehalt von 1 bis 5 mmol NH₂/g, einen Gehalt an Estergruppen von 1 bis 7 mmol/g, bevorzugt 2 bis 5 mmol/g, einen Gehalt an Amidgruppen von 1 bis 5 mmol/g, bevorzugt 1,5 bis 3,5 mmol/g, und eine NH-Funktionalität von 2 aus.

Die Viskosität der erfindungsgemäßen Verbindungen bei 23 °C variiert in einem weiten Bereich von 1 000 bis 10⁵ mPa·s.

Ein weiterer Gegenstand der vorliegenden Erfindung betrifft ein Verfahren zur Herstellung der erfindungsgemäßen Verbindungen, dadurch gekennzeichnet, daß Reaktionsprodukte aus diprimären Diaminen und Malein- oder Fumarsäureester (A) (Molverhältnis 1 : 2) mit 1 bis 4 mol primären und/oder sekundären Monoaminen bei 50 bis 150 °C, bevorzugt bei 80 bis 100 °C, in Substanz umgesetzt werden.

Die im erfindungsgemäßen Verfahren eingesetzten Ausgangsverbindungen (A) sind bekannt und werden in der EP 0 403 921 beschrieben. Die Umsetzung von (A) mit dem Monoamin erfolgt in Substanz bei 50 bis 150 °C, wobei beim Einsatz von primären Monoaminen bei Temperaturen von 80 bis 100 °C gearbeitet wird, da bei höheren Temperaturen mit Bildung cyclischer Imide gerechnet werden muß. Der sich bildende Alkohol wird bereits während der Reaktion aus dem Reaktionsprodukt abdestilliert. Bei den Monomeren handelt es sich entweder um primäre Monoamine, wie z. B. Butylamin, Hexylamin, 2-Ethylhexylamin, Decylamin, Dodecylamin, Cyclohexylamin, 2-Ethylhexoxypropylamin, oder um sekundäre Monoamine, wie z. B. Dibutylamin, Dihexylamin, Di-2-ethylhexylamin.

Die erfindungsgemäßen Verbindungen lassen sich problemlos lösemittelfrei bzw, in inerten Lösemitteln mit Polyisocyanaten zu 2K-PUR-Lacken verarbeiten. Das bedeutendste Anwendungsgebiet der erfindungsgemäßen Verbindungen ist ihre Verwendung als Reaktionskomponente für die Herstellung von 2K-PUR-Klebstoffen.

### Experimenteller Teil

### 1. Allgemeine Herstellungsvorschrift für die erfindungsgemäßen Verbindungen

### a) Herstellung der Ausgangsverbindung A

Zu dem auf 40 °C erhitzten Maleinsäureester wird das Diamin (H₂N-R-NH₂) so zudosiert, daß die Temperatur nicht über 60 °C steigt. Nach Beendigung der Diaminzugabe wird zur Vervollständigung der Reaktion noch ca. 3 h bei 60 °C weitererhitzt.

### b) Umsetzung von A mit Monoaminen

A wird mit dem Monoamin in dem gewünschten Molverhältnis bei 50 bis 150 °C zusammen so lange erhitzt, bis pro Mol eingesetztem Monoamin sich ein Mol Amid gebildet hat. Dies läßt sich durch Bestimmung des Amingehaltes des Reaktionsproduktes problemlos verfolgen. Die Reaktionstemperatur hängt sehr von der Struktur des eingesetzten Monoamins ab. Bei den primären Monoaminen hat sich 80 °C bis maximal 100 °C bewährt, bei den reaktionsträgeren sekundären Monoaminen muß bei 130 bis 150 °C gearbeitet werden. Der bei der Reaktion gebildete Alkohol wird nach der Reaktion abdestilliert. Dies ist dann der Fall, wenn es sich um höhersiedende Alkohole handelt; die freiwerdenden niedrigsiedenden Alkohole werden bereits während der Reaktion kontinuierlich abdestilliert.

Eingesetzte Diamin-Maleinsäureester (1 : 2)-Addukte:
A 1: 1 mol TMD + 2 mol Maleinsäurediethylester
A 2: 1 mol Hexamethylendiamin + 2 mol Maleinsäuredibutylester
A 3: 1 mol 2-Methylpentamethylendiamin + 2 mol Maleinsäuredi-2-ethylhexylester

| **Beispiel- Nr.** | **Reaktanten** | | **NH**_{**2**} **[mmol/g]** | **Viskosität (23 °C) [mPa·s]** |
|---|---|---|---|---|
| | **molA** | **mol Monoamin/Diamin** | | |
| 1 | 1 A 1 | 1 C₈H₁₇-NH₂ | 3,3 | 32·10³ |
| 2 | 1 A 1 | 1 (C₆H₁₃)₂-NH | 3,0 | 25·10³ |
| 3 | 1 A 2 | 1 C₆H₁₃-NH₂ | 3,2 | 63·10³ |
| 4 | 1 A 3 | 2 C₈H₁₇-O-(CH₂)₃-NH₂ | 1,8 | 28.10³ |

## Patentansprüche

1. Ester- und amidgruppenhaltige Polyamine,
durch gekennzeichnet,
daß sie mindestens eine Amidgruppe enthalten und folgende Zusammensetzung besitzen 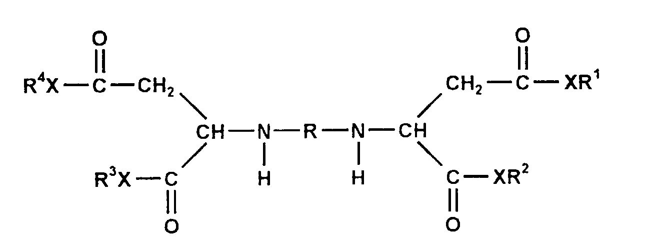 und die Substituenten die nachstehende Bedeutung haben:
R ist ein linearer, verzweigter oder cyclischer Kohlenwasserstoffrest mit 4 bis 18 C-Atomen,
R¹, R², R³, R⁴ sind gleiche oder verschiedene lineare, verzweigte oder cyclische Alkylreste oder gleich Wasserstoff,
X repräsentiert 0 bis 3 Sauerstoffatome und 1 bis 4 Stickstoffatome.

2. Polyamine nach Anspruch 1,
dadurch gekennzeichnet,
daß die cyclischen Kohlenwasserstoffreste der Substituenten ebenfalls am Ring weitere Alkylgruppen wie z. B. Methyl, Ethyl, Propyl, Butyl, aufweisen können.

3. Polyamine nach Anspruch 1 oder 2,
dadurch gekennzeichnet,
daß die Substituenten R, R¹, R², R³ und/oder R⁴ ein oder mehrere Sauerstoffatome anstelle einer CH₂-Gruppe aufweisen.

4. Polyamine nach den Ansprüchen 1 bis 3,
dadurch gekennzeichnet,
daß R Kohlenwasserstoffreste darstellt, die aus den folgenden Diaminen ausgewählt werden können: 1.4-Diaminobutan, 1.6-Diaminohexan, 2-Methylpentamethylendiamin, 2.2.4(2.4.4)-Trimethyl-1.6-diaminohexan, Isophorondiamin (IPD), 4.4'-Diaminodicyclohexylmethan oder 3.3'-Dimethyl-4.4'-diaminodicyclohexylmethan.

5. Polyamine nach den Ansprüchen 1 bis 4,
dadurch gekennzeichnet,
daß die Reste R' bis R⁴ Kohlenwasserstoffreste darstellen, die aus den folgenden Aminen ausgewählt werden können: Butylamin, Hexylamin, 2-Ethylhexylamin, Decylamin, Dodecylamin, Cyclohexylamin, 2-Ethylhexoxypropylamin, Dibutylamin, Dihexylamin, Di-2-ethylhexylamin, oder Alkylreste von Malein- oder Fumarsäureestern.

6. Verfahren zur Herstellung erfindungsgemäßer Verbindungen,
dadurch gekennzeichnet,
daß Reaktionsprodukte aus diprimären Diaminen und Malein- oder Fumarsäureester (A) (Molverhältnis 1 : 2) mit 1 bis 4 mol primären und/oder sekundären Monoaminen bei 50 bis 150 °C, bevorzugt bei 80 bis 100 °C, in Substanz umgesetzt werden.

7. Verwendung der Polyamine gemäß den Ansprüchen 1 bis 5 als Reaktionskomponente für 2K-PUR-Klebstoffe.
